# EUROPÄISCHE PATENTSCHRIFT

(11) **EP 1 114 638 B1**
(45) Veröffentlichungstag und Bekanntmachung des Hinweises auf die Patenterteilung: **16.03.2005**
(21) Anmeldenummer: 00126780.6
(22) Anmeldetag: 06.12.2000
(51) Int. Cl.: A61K 7/48, A61K 7/00, D21H 17/14, D06M 13/224, C14C 9/00, A61K 45/06

(54) **Lipidische Zusammensetzung und deren Verwendung zur Herstellung von kosmetischen/pharmazeutischen Mitteln, Textilien, Papier, Faserstoffen oder Leder**
Lipidic composition and its use for the preparation of cosmetic/pharmaceutic compositions, textiles, paper, fibers or leather
Composition lipidique et utilisation pour la preparation de compositions cosmétiques/pharmaceutiques, tissues, papier, fibres ou cuir

(30) Priorität: 06.12.1999 DE 19958612
(43) Veröffentlichungstag der Anmeldung: 11.07.2001
(73) Patentinhaber: Hübner, Georg, 40599 Düsseldorf (DE)
(72) Erfinder: Hübner, Georg, 40599 Düsseldorf (DE)
(74) Vertreter: Ackmann, Günther, Dr.-Ing.

(56) Entgegenhaltungen:
- DE-A- 19 832 122
- US-A- 5 683 706
- US-A- 6 153 209

## Beschreibung

Die Erfindung betrifft eine lipidische Zusammensetzung, umfassend zumindest eine lipidhaltige Substanz ausgewählt aus der Gruppe bestehend aus: Argan-Öl, unverseifbarer Anteil von Argan-Öl, unverseifbarer Anteil von Avocado-Öl, diesbezüglich naturidentische Substanzen, Tocopheryl-Linoleat, lipidhaltige Substanzen isoliert aus Theobroma cacao (Kakao), Garcinia indica (Kokum), Shorea stenoptera (Illipe), Shorea robusta (Sal), Mangifera indica (Mango) und mindestens ein Lösungsmittel, sowie die Verwendung von Neopentansäureester, n-Octadecansäure-12-[(1-oxooctadecyl)oxy]-ester oder einer Kombination aus beiden als Lösemittel für die oben genannten lipischen Zusammensetzungen.

Lipidhaltige Mittel finden insbesondere in der kosmetischen und pharmazeutischen Industrie in vielfältiger Form Anwendung. Vor allem bei schwer löslichen Substanzen mit hohem Fettanteil ist es schwierig, diese in eine anwendbare und wirksame Form zu bringen. Dabei besteht insbesondere bei vielen Pflanzenextrakten, beispielsweise sogenannten "Exoticbutters", das Problem, dass diese eine schmalzartige, kristalline Konsistenz aufweisen und kaum in einen homogenen, flüssigen Zustand gebracht werden können. Solche Extrakte sind darüber hinaus temperaturempfindlich, d.h. sie neigen bei hohen Temperaturen zur Entmischung und bei niedrigen Temperaturen zur Kristallisation. Auf Grund dieser fortschreitenden Inhomogenität sind solche Produkte daher nicht nur in ihrer Verwendung eingeschränkt, sondern auch schwer zu vermarkten, da die Produktästhetik deutlich reduziert ist. Viele lipidische Wirkstoffe können ferner beispielsweise in kosmetischen oder pharmazeutischen Mitteln ihre optimale Wirksamkeit nur entfalten, wenn sie in gelöster oder emulgierter Form vorliegen.

Der Erfindung liegt daher die Aufgabe zugrunde, eine lipidische Zusammensetzung bereitzustellen, die eine Verwendung von lipidhaltigen Substanzen in dauerhaft homogener Konsistenz bei verbesserter Anwendbarkeit und Wirksamkeit gewährleistet.

Erfindungsgemäß wird die Aufgabe dadurch gelöst, dass als Lösungsmittel Neopentansäureester, n-Octadecansäure-12-[(1-oxo-octadecyl)oxy]-ester oder eine Kombination aus beiden enthalten ist.

Mit Hilfe dieser Lösungsmittel ist es erstmals gelungen, die schwer löslichen und zur Inhomogenität neigenden, lipidhaltigen Substanzen zu verflüssigen und eine dauerhafte Homogenität zu gewährleisten. Dabei können die Lösungsmittel einzeln oder in beliebiger Kombination eingesetzt werden. Insbesondere hochlipidhaltige Pflanzenextrakte, beispielsweise sogenannte "Exoticbutters", die eine schmalzartige Konsistenz aufweisen, nicht pumpbar sind und in Abhängigkeit von der Temperatur zur Inhomogenität neigen, können in der erfindungsgemäßen Zusammensetzung gelöst werden. Es entsteht ein homogenes Produkt, welches sowohl bei Raumtemperatur als auch bei niedrigen Temperaturen flüssig bleibt und pumpbar ist. Durch die deutliche Erhöhung der thermischen Stabilität wird darüber hinaus ein Entmischen bei Temperaturen über 25° C vermieden, so dass sich auch bei längerer Lagerung kein Öl an der Oberfläche abscheidet, was die Produktästhetik dauerhaft verbessert. Darüber hinaus wird bei niedrigen Temperaturen ein Auskristallisieren von Bestandteilen der Zusammensetzung durch die genannten Lösungsmittel verhindert. Die lipidische Zusammensetzung zeichnet sich ferner durch eine hohe Spreitfähigkeit aus, so dass sie beispielsweise auch sprühbar ist und in kosmetischen oder pharmazeutischen Sprays eingesetzt werden kann. Auf Grund dieser vorteilhaften Eigenschaften kann die lipidische Zusammensetzung sehr gut verarbeitet werden, so dass eine vielseitige Anwendbarkeit gegeben ist. Das erfindungsgemäße Produkt eignet sich dabei sowohl zum Beimischen als Bestandteil anderer Mittel als auch zum alleinigen Einsatz als reines Produkt. Durch die flüssige Konsistenz und die hohe thermische Stabilität ist die lipidische Zusammensetzung beispielsweise sehr gut auf der Haut verteilbar, so dass eine einfache Handhabung bei hoher Wirksamkeit der Inhaltsstoffe gewährleistet ist. Generell sind bei der erfindungsgemäßen Zusammensetzung die therapeutisch wirksamen Inhaltsstoffe auf Grund des Vorliegens einer homogenen Lösung in einer hochwirksamen Form applizierbar. Die Lösungsmittel, die sich zu diesem Zweck überraschender Weise als besonders vorteilhaft erwiesen haben, stellen einzeln oder in Kombination die oben genannten positiven Eigenschaften sicher.

Neopentansäureester verhindern ein Auskristallisieren der gelösten Substanzen bei niedrigen Temperaturen, so dass diese neben ihrer Eigenschaft als Lösungsmittel auch wesentlich zur Kältestabilität des Produktes beitragen. Als besonders vorteilhaft haben sich dabei Isodecyl-Neopentanoat und Tridecyl-Neopentanoat erwiesen. Die n-Octadecansäure-12-[(1-oxooctadecyl)oxy]-ester haben sich schließlich als Lösungsmittel herausgestellt, welche für ein sehr breites Spektrum an lipidhaltigen Substanzen, insbesondere Pflanzenextrakten, geeignet sind. Insbesondere n-Octadecansäure-12-[(1-oxooctadecyl)oxy]-2-octyldodecyl-ester (Octyldodecylstearoylstearat) und n-Octadecansäure-12-[(1-oxooctadecyl)oxy]-2-isohexadecyl-ester (lsocetylstearoylstearat) sind hervorragende Lösungsmittel für viele hochlipidhaltige Substanzen, wobei sie darüber hinaus eine dauerhafte Homogenität und hohe thermische Stabilität der Lösung gewährleisten. Bisher wurde Isocetylstearoylstearat lediglich als Zusatzstoff in Haarpflegemitteln verwendet (beispielsweise in WO 9501152), so dass diese vorteilhaften Eigenschaften und der breite Anwendungsbereich überraschender Weise festgestellt wurden. Auf Grund der oben genannten Eigenschaften ist lsocetylstearoylstearat in besonders vorteilhafter Weise auch ausschließlich als Lösungsmittel in der lipidischen Zusammensetzung einsetzbar. Octyldodecylstearoylstearat ist bisher als Inhaltsstoff von kosmetischen Produkten, beispielsweise Puder (vgl. WO 9704737), verwendet worden. Auch hier ergab sich die vorteilhafte Verwendbarkeit als Lösungsmittel für hochlipidhaltige Substanzen in überraschender Weise. Die beiden genannten Stearinsäure-stearoylester eignen sich besonders gut als Lösungsmittel für die erfindungsgemäße Zusammensetzung, es können jedoch auch andere Verbindungen aus dieser Gruppe eingesetzt werden.

Als lipidhaltige Substanzen sind in der enfindungsgemäßen Zusammensetzung insbesondere Pflanzenextrakte oder diesbezüglich naturidentische Substanzen enthalten. So kann die lipidische Zusammensetzung als lipidhaltige Substanz beispielsweise Avocadoöl und/oder Arganöl enthalten, welche sich auf Grund ihres hohen Gehaltes an Phytosterinen durch hautpflegende Eigenschaften auszeichnen. Dabei ist in einem besonders vortellhaften Ausführungsbeispiel vorgesehen, dass als lipidhaltige Substanz die unverseifbaren Anteile von Avocadoöl und/oder Arganöl oder diesbezügliche naturidentische Substanzen enthalten sind. Das Unverseifbare ist derjenige Anteil der Öle, welcher bei der Gewinnung von Fettsäuren einer Verseifung widersteht. Der unverseifbare Anteil von Arganöl, welches aus der Argannuss gewonnen wird, enthält besondere Phytosterine in hohen Konzentrationen, die der Substanz hautpflegende und entzündungshemmende Eigenschaften verleihen. Der unverseifbare Anteil von Avocadoöl enthält insbesondere hohe Konzentrationen an Phytosterinen, mehrfach ungesättigten Fettsäuren und den Vitaminen E und F. Neben hautpflegenden Eigenschaften hat der unverseifbare Anteil von Avocadoöl vor allem antiphlogistische, antirheumatische, antimykotische, antiscabiotische und schmerzstillende sowie juckreizhemmende Eigenschaften. Diese Substanz ist also vielseitig anwendbar und auf Grund seiner hohen Konzentration an Wirkstoffen ein therapeutisch äußerst wirksamer Naturstoff. Auf Grund ihrer zähen und schwer verteilbaren Konsistenz waren die unverseifbaren Anteile der genannten Öle aber bisher sehr schwierig zu verarbeiten und daher nur eingeschränkt nutzbar. Innerhalb der erfindungsgemäßen Zusammensetzung sind das Unverseifbare des Avocadoöls und/oder Arganöls nun aber in flüssiger, homogener und leicht zu verarbeitender Qualität verfügbar, so dass die vorteilhaften Eigenschaften dieser hochlipidhaltigen Substanzen optimal genutzt werden können. Statt des Pflanzenextraktes kann dabei auch eine diesbezüglich naturidentische Substanz mit gleichen Eigenschaften verwendet werden.

Als lipidhaltige Substanz kann ferner in vorteilhafter Weise Tocopheryl-Linoleat in der lipidischen Zusammensetzung enthalten sein. Diese Substanz, die aus Vitamin E und Linolsäure besteht, eignet sich besonders als Hautpflegemittel. Als Bestandteil der erfindungsgemäßen Zusammensetzung ist es sehr leicht auf der Haut verteilbar und kann daher seine Wirksamkeit besonders gut entfalten. Das Gleiche gilt auch für sogenannte ,Exoticbutters", die ebenfalls im kosmetischen Bereich als Hautpflegesubstanzen eingesetzt werden. Daher kann die lipidhaltige Substanz auch in vorteilhafter Weise aus Pflanzen isoliert sein. Als exotische Pflanzen kommen hierbei beispielsweise Theolroma ccao (Cocoa), Garcimia indica (Kokum), Sherca stenopfera (Illipe), Shearea robusta (SAL), Butyrospermum partric (Shea) oder Magnifera indica (Mango) in Betracht.

In vorteilhafter Ausgestaltung der Erfindung ist vorgesehen, dass die lipidhaltige Substanz in einer Konzentration von 0,05 bis 50 Gew.-% enthalten ist. Zusätzlich kann die lipidische Zusammensetzung Vitamine, beispielsweise Vitamin E und F, und/oder mehrfach ungesättigte Fettsäuren enthalten. Darüber hinaus können zusätzlich übliche Zusatz- und Hilfsstoffe zugegeben werden, um dem Produkt die gewünschte Konsistenz zu verleihen bzw. eine anwendbare Qualität zu einzustellen.

Auf Grund der Vielzahl der möglichen lipidhaltigen Substanzen, die in der erfindungsgemäßen Zusammensetzung gelöst sein können, sind vielfältige vorteilhafte Verwendungen der lipidischen Zusammensetzung möglich. Ein bevorzugter Anwendungsbereich liegt dabei in der Verwendung der lipidischen Zusammensetzung als kosmetisches und/oder pharmazeutisches Mittel oder als Bestandteil derselben. Die Zusammensetzung kann dabei beispielsweise in Form von Salben, Cremes, Ölen, Lotionen oder dergleichen angewendet werden. Auf Grund der flüssigen Konsistenz mit hoher Spreitfähigkeit ist die erfindungsgemäße Zusammensetzung aber auch in besonderes vorteilhafter Weise in sprühbarer Form als Spray anwendbar. Hierdurch können die Wirkstoffe besonders gut auf der Haut verteilt werden, so dass die Wirksamkeit der Inhaltsstoffe gesteigert werden kann. Möglich ist ferner die Verwendung der lipidischen Zusammensetzung zur Herstellung von Textilien, Papier, Faserstoffen, beispielsweise Kosmetik- oder Pflegetüchern, oder Leder. Hierbei kann die lipidische Zusammensetzung insbesondere zur Veredelung oder zur Benetzung der genannten Materialien mit Wirkstoffen dienen.

Im folgenden sind einige Beispiele von erfindungsgemäßen Zusammensetzungen aufgeführt, wobei es sich hierbei aber weder um bevorzugte Ausführungsformen noch um eine abschließende Aufzählung handelt. Die folgenden Beispiele dienen vielmehr ausschließlich der Erläuterung der erfindungsgemäßen Zusammensetzung und sind daher nicht einschränkend zu verstehen.

| | | |
|---|---|---|
| Beispiel 1 | Avocadoöl, unverseifbares | 50 % |
| | Isocetylstearoylstearat | 30 % |
| | di-n-Octylether | 10 % |
| | Isodecyl-Neopentanoat | 10 % |
| Beispiel 2 | Tocopheryl-Linoleat | 40 % |
| | Isocetylstearoylstearat | 40 % |
| | Isodecyl-Neopentanoat | 10 % |
| | Zusatz- und Hilfsstoffe | 10 % |
| Beispiel 3 | Tocopheryl-Linoleat | 30 % |
| | Mangoextrakt | 30 % |
| | Isocetylstearoylstearat | 20 % |
| | di-n-Octylether | 10 % |
| | Zusatz- und Hilfsstoffe | 10 % |
| Beispiel 4 | Arganöl, unverseifbares | 40 % |
| | Kokumextrakt | 25 % |
| | Octyldodecylstearoylstearat | 30 % |
| | Tridecyl-Neopentanoat | 5 % |
| (alle Konzentrationsangaben in Gew.-%). | | |

## Patentansprüche

1. Verwendung von Neopentansäureester, n-Octadecansäure-12-[(1-oxooctadecyl) oxy]-ester oder einer Kombination aus beiden als Lösemittel für lipidische Zusammensetzungen, umfassend zumindest eine lipidhaltige Substanz ausgewählt aus der Gruppe bestehend aus: Argan-Öl, unverseifbarer Anteil von Argan-Öl, unverseifbarer Anteil von Avocado-Öl, diesbezüglich naturidentische Substanzen, Tocopheryl-Linoleat, lipidhaltige Substanzen isoliert aus Theobroma cacao (Kakao), Garcinia indica (Kokum), Shorea stenoptera (Illipe), Shorea robusta (Sal), Mangifera indica (Mango).

2. Lipidische Zusammensetzung, umfassend zumindest eine lipidhaltige Substanz ausgewählt aus der Gruppe bestehend aus: Argan-Öl, unverseifbarer Anteil von Argan-Öl, unverseifbarer Anteil von Avocado-Öl, diesbezüglich naturidentische Substanzen, Tocopheryl-Linoleat, lipidhaltige Substanzen isoliert aus Theobroma cacao (Kakao), Garcinia indica (Kokum), Shorea stenoptera (IIIipe), Shorea robusta (Sal), Mangifera indica (Mango) und mindestens ein Lösungsmittel
**dadurch gekennzeichnet,**
**dass** als Lösungsmittel Neopentansäureester, n-Octadecansäure-12-[(1-oxo-octadecyl)oxy]-ester oder eine Kombination aus beiden enthalten ist.

3. Lipidische Zusammensetzung nach Anspruch 2,
**dadurch gekennzeichnet,**
**dass** der Octadecansäureester n-Octadecansäure-12-[(1-oxooctadecyl)oxy]-octyldodecyl-ester oder n-Octadecansäure-12-[(1-oxooctadecyl)oxy]-isohexa-decyl-ester und/oder der Neopentansäureester Isodecyl-Neopentanoat oder Tridecyl-Neopentanoat ist.

4. Lipidische Zusammensetzung nach einem oder mehreren der Ansprüche 2 bis 4,
**dadurch gekennzeichnet,**
**dass** die lipidhaltige Substanz in einer Konzentration von 0,05 bis 50 Gew.-% enthalten ist.

5. Lipidische Zusammensetzung nach einem oder mehreren der Ansprüche 2 bis 4,
**dadurch gekennzeichnet,**
**dass** die lipidische Zusammensetzung zusätzlich Vitamine, beispielsweise Vitamin E und F, und/oder mehrfach ungesättigte Fettsäuren enthält.

6. Lipidische Zusammensetzung nach einem oder mehreren der Ansprüche 2 bis 5,
**dadurch gekennzeichnet,**
**dass** die lipidische Zusammensetzung zusätzlich übliche Zusatz- und Hilfsstoffe enthält.

7. Verwendung der lipidischen Zusammensetzung nach einem oder mehreren der Ansprüche 2 bis 6 zur Herstellung eines kosmetischen und/oder pharmazeutischen Mittels.

8. Verwendung der lipidischen Zusammensetzung nach Anspruch 7 als Spray.

9. Verwendung der lipidischen Zusammensetzung nach einem oder mehreren der Ansprüche 2 bis 6 zur Herstellung von Textilien.

10. Verwendung der lipidischen Zusammensetzung nach einem oder mehreren der Ansprüche 2 bis 6 zur Herstellung von Papier.

11. Verwendung der lipidischen Zusammensetzung nach einem oder mehreren der Ansprüche 2 bis 6 zur Herstellung von Faserstoffen, insbesondere Kosmetik- oder Pflegetüchern.

12. Verwendung der lipidischen Zusammensetzung nach einem oder mehreren der Ansprüche 2 bis 6 zur Herstellung von Leder.

## Claims

1. Use of neopentanoic ester, n-octadecanoic acid 12-[(1-oxooctadecyl)oxy] ester or a combination of the two as a solvent for lipidic compositions, comprising at least one lipid-containing substance selected from the group consisting of: argan oil, an unhydrolyzable fraction of argan oil, an unhydrolyzable fraction of avocado oil, nature-identical substances in this respect, tocopheryl linoleate, lipid-containing substances isolated from Theobroma cacao (cocoa), Garcinia indica (kokum), Shorea stenoptera (illipe), Shorea robusta (sal) and Mangifera indica (mango).

2. Lipidic composition, comprising at least one lipid-containing substance selected from the group consisting of: argan oil, an unhydrolyzable fraction of argan oil, an unhydrolyzable fraction of avocado oil, nature-identical substances in this respect, tocopheryl linoleate, lipid-containing substances isolated from Theobroma cacao (cocoa), Garcinia indica (kokum), Shorea stenoptera (illipe), Shorea robusta (sal) and Mangifera indica (mango) and at least one solvent, **characterized in that** neopentanoic ester, n-octadecanoic acid 12-[(1-oxooctadecyl)oxy] ester or a combination of the two is contained as the solvent.

3. Lipidic composition according to Claim 2, **characterized in that** the octadecanoic ester is 12-[(1-oxooctadecyl)oxy]octyldodecyl n-octadecanoate or 12-[(1-oxooctadecyl)oxy]isohexadecyl n-octadecanoate and/or the neopentanoic ester is isodecyl neopentanoate or tridecyl neopentanoate.

4. Lipidic composition according to one or more of Claims 2 to 4, **characterized in that** the lipid-containing substance is contained in a concentration of 0.05 to 50% by weight.

5. Lipidic composition according to one or more of Claims 2 to 4,
**characterized in that** the lipidic composition additionally contains vitamins, for example vitamin E and F, and/or polyunsaturated fatty acids.

6. Lipidic composition according to one or more of Claims 2 to 5, **characterized in that** the lipidic composition additionally contains customary additives and auxiliaries.

7. Use of the lipidic composition according to one or more of Claims 2 to 6 for the preparation of a cosmetic and/or pharmaceutical agent.

8. Use of the lipidic composition according to Claim 7 as spray.

9. Use of the lipidic composition according to one or more of Claims 2 to 6 for the production of textiles.

10. Use of the lipidic composition according to one or more of Claims 2 to 6 for the production of paper.

11. Use of the lipidic composition according to one or more of Claims 2 to 6 for the production of fibres, in particular cosmetic or personal hygiene tissues.

12. Use of the lipidic composition according to one or more of Claims 2 to 6 for the production of leather.

## Revendications

1. Utilisation d'ester d'acide néopentanoïque, de 12-[(1-oxooctadécyl)oxy]ester d'acide n-octadécanoïque ou une combinaison des deux comme solvant pour des compositions lipidiques, comprenant au moins une substance contenant des lipideschoisie parmi le groupe consistant en l'huile d'argan, la partie non saponifiable de l'huile d'argan, la partie non saponifiable de l'huile d'avocat, des substances de natures identiques à celles-ci, le linoléate de tocophéryle, des substances contenant des lipides isolées de *Thoebroma cacao* (cacao), de *Garcinia indica* (kokum), de *Shorea stenoptera* (illipé), de *Shorea robusta* (sal), de *Magnera indica* (mangue).

2. Composition lipidique, comprenant au moins une substance contenant des lipides choisie parmi le groupe consistant en l'huile d'argan, la partie non saponifiable de l'huile d'argan, la partie non saponifiable de l'huile d'avocat, des substances de natures identiques à celles-ci, le linoléate de tocophéryle, des substances lipidiques isolées de *Thoebroma cacao* (cacao), de *Garcinia indica* (kokum), de *Shorea stenoptera* (illipé), de *Shorea robusta* (sal), de *Magnifera indica* (mangue), et au moins un solvant, **caractérisée en ce que** comme solvant est présent l'ester d'acide néopentanoïque, le 12-[(1-oxooctadécyl)oxy]ester d'acide n-octadécanoïque ou une combinaison des deux.

3. Composition lipidique selon la revendication 2, **caractérisée en ce que** l'ester d'acide octadécanoïque est le 12-[(1-oxooctadécyl)oxy]octyldodécylester d'acide n-octadécanoïque ou le 12-[(1-oxooctadécyl)oxy]isohexadécylester d'acide n-octadécanoïque et/ou l'ester d'acide néopentanoïque est le néopentanoate d'isodécyle ou le néopentanoate de tridécyle.

4. Composition lipidique selon l'une ou plusieurs des revendications 2 à 4, **caractérisée en ce que** la substance contenant des lipides est présente en une concentration allant de 0,05 à 50% en poids.

5. Composition lipidique selon l'une ou plusieurs des revendications 2 à 4, **caractérisée en ce que** la composition lipidique contient en outre, des vitamines, par exemple les vitamines E et F, et/ou des acides gras plusieurs fois insaturés.

6. Composition lipidique selon l'une ou plusieurs des revendications 2 à 5, **caractérisée en ce que** la composition lipidique contient en outre, des additifs et auxiliaires usuels.

7. Utilisation de la composition lipidique selon l'une ou plusieurs des revendications 2 à 6, pour la préparation d'un agent cosmétique et/ou pharmaceutique.

8. Utilisation de la composition lipidique selon la revendication 7, comme un spray.

9. Utilisation de la composition lipidique selon l'une ou plusieurs des revendications 2 à 6, pour la préparation de textiles.

10. Utilisation de la composition lipidique selon l'une ou plusieurs des revendications 2 à 6, pour la préparation de papier.

11. Utilisation de la composition lipidique selon l'une ou plusieurs des revendications 2 à 6, pour la préparation de substances fibreuses, en particulier de serviettes cosmétiques ou de soin.

12. Utilisation de la composition lipidique selon l'une ou plusieurs des revendications 2 à 6, pour la préparation de cuir.
